# EUROPEAN PATENT APPLICATION

(11) **EP 4 800 026 A1**
(43) Date of publication of application: **02.09.2026**
(21) Application number: 25185494.9
(22) Date of filing: 26.06.2025
(51) Int. Cl.: C07K 14/37, A23L 33/00, A61K 8/9728, A61K 38/16

(54) **ANTIMICROBIAL PEPTIDE VARIANTS AND USES THEREOF**

(30) Priority: 28.02.2025 US 202519067144
(71) Applicant: Antinbio, Inc., Worcester, MA 01605 (US)
(72) Inventor: LUO, Ke, Holden, 01520 (US)
(74) Representative: Eisenführ Speiser

(57) **Abstract**

Antimicrobial peptides are provided. In particular, the antimicrobial peptides having the amino acid sequence selected from SEQ ID NOs: 2-21. The antimicrobial peptide has antimicrobial activity against Gram-negative bacteria, and independently, against Gram-positive bacteria. A composition, pharmaceutical composition, food additive, cosmetic composition, or hygine product having an antimicrobial peptide including an amino acid sequence selected from SEQ ID NOs: 2-21 is also provided. A method of treating an infectious disease caused by bacteria including a step of administering a pharmaceutical composition having the antimicrobial peptide as an active ingredient is also provided.

## Description

### SEQUENCE LISTING

The instant application contains a Sequence Listing which has been submitted electronically in Sequence Listing XML format and is hereby incorporated by reference in its entirety. Said Sequence Listing XML copy, created on February 27, 2025, is named J4069-00301_SL.xml and is 30,980 bytes in size.

### FIELD OF THE INVENTION

The disclosure relates generally to variants of an antimicrobial peptide derived from fungi, antimicrobial compositions containing one or more of the variants, and methods of using the same to inhibit microbial infections in a subject and to promote animal growth.

### BACKGROUND

Antimicrobial peptides, also known as host defense peptides, are a class of low-molecular-weight (typically 6 to 100 amino acids) polypeptides that can exhibit broad-spectrum antimicrobial activity against different pathogens including: gram-negative bacteria, gram-positive bacteria, mycobacteria, viruses, fungi, while having little to no effect on the cells of the treated animals. Compared with conventional antibiotics, one of the strengths of antimicrobial peptides is their low propensity for resistance development for reasons related to both their mechanisms of killing, and their role in innate immunity modulation. Such peptides are also typically highly water-soluble. Antimicrobial peptides are generally induced by external conditions and encoded by certain genes of various biological cells, and play an important role in natural immune defense system.

Fungi are an important source of antimicrobial peptides. Fungal defensin peptides such as Plectasin (isolated from the mushroom *Pseudoplectania nigrella*) have high activity against several methicillin-resistant *S. aureus* specie. Defensins are considered part of the innate immune response, and act mainly by disrupting the structure of bacterial cell membranes. Defensin-like peptides, such as plectasin, exhibit activity against several methicillin-resistant strains of *Staphylococcus aureus.* Hegrisin, a defensin-like antimicrobial peptide from a fungal genomic sequence of *Helicocarpus griseus,* has strong activity against multiple Gram-positive bacteria.

In recent years, Spotty liver diseases (SLD) caused by *Campylobacter hepaticus* and necrotic *enteritis* caused by *Clostridium perfringens* have reemerged in USA, Australia and Europe following antibiotics bans. SLD is a serious infectious disease that affects layer chickens causing up to 10% flock mortalities and a 25% reduction in egg production. Treatment becomes increasingly difficult because these bacteria are resistant to multiple antibiotics. It is urgent to develop novel antimicrobial compounds, especially new compounds having bactericidal ability to drug-resistant bacteria, such as *C*. *hepaticus, C. perfringens* and methicillin-resistant *S. aureus* (MRSA).

### SUMMARY

In various embodiments, the present disclosure describes one or more antimicrobial peptides that exhibit exceptional activity against selected Gram negative bacteria, selected Gram negative bacteria, low to no host toxicity, and high level expression in a recombinant expression system.

In view of the foregoing disadvantages inherent to most of the known types of Antimicrobial peptides present in the prior art, the present invention describes novel Antimicrobial peptides with exceptional killing activity against Gram negative and Gram positive bacteria, low to no host toxicity, and high level expression in a recombinant expression system.

In some aspects, this disclosure provides for peptides and compositions comprising such to prevent or treat animals against bacterial infection. Other than manufacture ease and price, Antimicrobial peptides have many advantages over conventional antibiotics mentioned heretofore as well as improved efficiency that is not anticipated, rendered obvious, suggested, or even implied by any of the prior art antimicrobial peptides, either alone or in any combination thereof. To attain this, the present invention generally comprises a 38 amino acid sequence that is introduced into animals as the peptide, or as part of a polypeptide, or expressed from a DNA or RNA oligonucleotide. An example of this peptide will be presented and is referred to as Hegrisin.

There has thus been outlined, rather broadly, the more important features of the invention in order that the detailed description thereof may be better understood, and in order that the present contribution to the art may be better appreciated. There are additional features of the invention that will be described hereinafter.

In some aspects, this disclosure provides for an antimicrobial peptide that will overcome the shortcomings of the prior art antibiotics.

In some aspects, this disclosure provides for an antimicrobial peptide that has exceptional killing against bacteria. The antimicrobial peptide is safe to animal to which it is administered.

In some aspects, this disclosure provides for an antimicrobial peptide consisting, comprising, or consisting essentially of, an amino acid sequence having the following sequence:
GX₁X₂CX₃X₄X₅X₆X₇X₈DX₉X₁₀CHX₁₁HCKSIX₁₂GYX₁₃GGYCKX₁₄GGX₁₅CKCY (SEQ ID NO: 22) (Formula I),
wherein X₁ is F or W;
X₂ is G or S;
X₃ is T, N or G;
X₄ is I or F;
X₅ is W or F;
X₆ is G or N;
X₇ is G or E;
X₈ is N or D;
X₉ is E, L, or D;
X₁₀ is P or R;
X₁₁ is R, Q, N, or K;
X₁₂ is K or R;
X₁₃ is K or R;
X₁₄ is V, F, or L; and
X₁₅ is V or I.

In some aspects, X₆ is G, X₇ is G, and X₈ is N.

In some aspects, the amino acid sequence is selected from the group consisting of SEQ ID NOs: 1-21.

In some aspects, the antimicrobial peptide can have antimicrobial activity against Gram-negative bacteria. The antimicrobial peptide can have antimicrobial acitivity against the Gram- negative bacteria of *Campylobacter hepaticus* or *Campylobacter jejuni,* including any subspecies or strains thereof.

In some aspects, the antimicrobial peptide can have antimicrobial activity against Gram-positive bacteria. The antimicrobial peptide can have antimicrobial acitivity against the Gram- positive bacteria of *Clostridium perfringens, Staphylococcus aureus, Staphylococcus aureus* MRSA, *Staphylococcus epidermidis,* or *Bacillus subtilis,* including any subspecies or strains thereof.

In some aspects, this disclosure provides for an antimicrobial peptide composition comprising the antimicrobial peptide of Formula I as an active ingredient. The antimicrobial peptide composition can be a pharmaceutical composition.

In some aspects, this disclosure provides for an antimicrobial food additive comprising the antimicrobial peptide of Formua I as an active ingredient.

In some aspects, this disclosure provides for a cosmetic composition comprising the antimicrobial peptide of Formula I as an active ingredient.

In some aspects, this disclosure provides for a hygiene product comprising the antimicrobial peptide of Formula I as an active ingredient.

In some aspects, this disclosure provides for a method of treating an animal having an infectious disease caused by bacteria, the method comprising administering a pharmaceutical composition comprising the antimicrobial peptide of Formula I as an active ingredient. The antimicrobial peptide can be the amino acid sequence of SEQ ID NO: 3, SEQ ID NO: 4, SEQ ID NO: 5, SEQ ID NO: 11, SEQ ID NO: 18, or SEQ ID NO: 21.

Other objects and advantages of the present invention will become obvious to the reader, and it is intended that these objects and advantages are within the scope of the present invention. To the accomplishment of the above and related objects, this invention may be embodied in the form illustrated in the accompanying drawings, attention being called to the fact, however, that the drawings are illustrative only, and that changes may be made in the specific construction illustrated.

### BRIEF DESCRIPTION OF THE FIGURES

The features and advantages of the antimicrobial peptide halymorin variants described herein will be more fully disclosed in, or rendered obvious by the following detailed description of the preferred embodiments, which are to be considered together with the accompanying drawings, wherein:

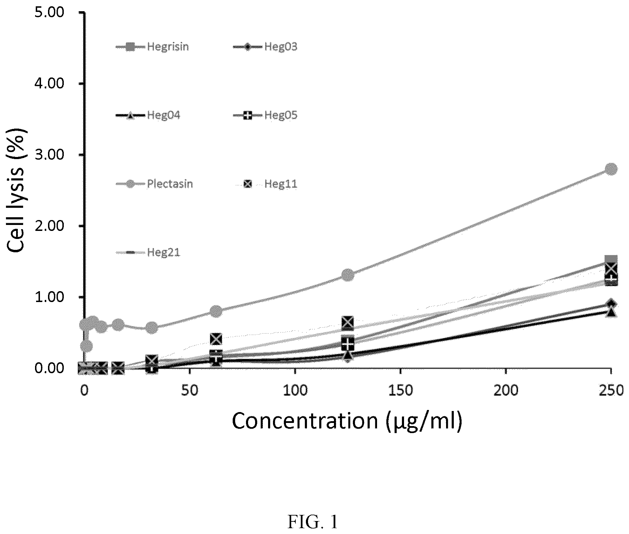

**FIG. 1** is a graph showing a safety profile based on a hemolysis assay for exemplary antimicrobial peptide Hegrisin variants.

### DETAILED DESCRIPTION

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of the description and should not be regarded as limiting.

The antimicrobial peptide variants of the present disclosure are based on hegrisin, which is a peptide obtained from the genomic sequence of the fungus, *Helicocarpus griseus.* Hegrisin contains 38 amino acids, has a molecular weight of 4111.8 Da, and a +6.2 positive charge. Hegrisin and its variants have three pairs of disulfide bonds and an antiparallel β-sheet, which fold into a cysteine-stabilized alpha-beta (CSαβ) structure, similar to the structure of many vertebrate and fungal β-defensins.

In some embodiments, peptide variants of hegrisin may comprise 20-100 amino acids. In some embodiments, the variants comprise 30-50, 35-50, 30-45, 30-40, or 35-45 amino acids or amino acid residues. All ranges are inclusive and combinable. In some embodiments, the hegrisin variants may consist of 38 amino acids or amino acid residues. In some embodiments, the hegrisin variants may comprise at least one amino acid sequence selected from the group consisting of SEQ ID NOs: 2-21.

In some embodiments, the N-terminal of the hegrisin pepetide variants may be acetylated or palmitoylated. If the N-terminal is acetylated, superior antimicrobial activity may be achieved and the peptide may be protected from proteolytic degradation. If the N-terminal is palmitoylated, then permeability into cells may be enhanced.

In some embodiments, an amino group (-NH₂) or a methyl group (-CH₃) is added to the C-terminal of the hegresin peptide variant. If the C-terminal of the hegresin peptide variant is amidated, resistance to proteases and positive net charge may be further enhanced. If the C-terminal is methylated, then *in vivo* stability may be increased based on an improved resistance to exopeptidases, which cleave the peptide from the terminal.

The relatively short peptide sequences of this disclosure afford several beneficial properties, including: commercial viability, strong inner membrane permeability, an absence of cytotoxicity, and minimal or no hemolytic activity. Short peptides are commercially viable because they can be either synthesized de novo on a commercial peptide synthesizer, or expressed in high yield in appropriate expression systems (e.g., fermentation). It was surprisingly discovered that the hegresin variant peptides of this disclosure are not toxic to certain host cells (e.g., yeast, including *Saccharomyces cerevisiae*)*,* which when combined with their relatively short length results in very high fermentation yields. In some embodiments, the peptides of this disclosure can be expressed in yeast at yields of 1-10 g/L. In some embodiments, the peptides of this disclosure can be expressed in yeast at a yield of up to 6 g/L.

The hegresin peptide variants of this disclosure can permeate membranes of microbes, including bacteria. Without being bound by theory, the hegresin peptide variants of this disclosure exhibit antimicrobial activity by directly permeating into the inner membrane of bacteria.

Medical practice often involves the administration of antibiotics to patients. Such treatments can involve administration of broad-spectrum antibiotics, or antibiotics that target many gram-positive bacterial species or many gram-negative species without discrimination, often resulting in undesireable side effects. Similarly, use of broad-spectrum antibiotics in farming and agriculture pose environmental concerns, including entry of such antibiotics into the human and animal food chain which may be deleterious to health and may add to development of microbial resistance. In some embodiments, this disclosure provides for Rather, in an example, selective targeting of a first microbial (eg, bacterial) species or strain of the microbiota.

The hegrisin peptide variants of the present disclosure were designed and prepared to exhibit antimicrobial activity against one or more pathogens selected from a group consisting of bacteria, such as Gram-positive bacteria (e.g., *Firmicutes*)*,* Gram-negative bacteria (e.g., *Gracilicutes*)*,* Gram-variable bacteria (e.g., *Mollicutes*)*,* uneven Gram stain (e.g., *Mendocutes*)*,* fungi, and molds. Gram-positive bacteria can include or exclude *Staphylococcus* and *Streptococcus,* and their strains. Gram-negative bacteria can include or exclude *Bacillus, Clostridia, Listeria, Corynebacterium, Campylobacter,* and their strains. In some embodiments, the hegrisin variants have antimicrobial activity against one or more bacteria, including *Campylobacter hepaticus, Campylobacter jujuni, Campylobacter coli, Campylobacter fetus, Campylobacter ureolyticus, Clostridium perfringens, Staphylococcus aureus, Staphylococcus epidermidis* and *Bacillus subtilis.*

In some embodiments, the hegresin peptide variants of this disclosure exhibit antimicrobial activity against a bacterial species selected from: a *Bacteroides* species selected from *caccae, capillosus, cellulosilyticus, coprocola, coprophilus, coprosuis, distasonis, dorei, eggerthii, faecis, finegoldii, fluxus, fragalis, intestinalis, melaninogenicus, nordii, oleiciplenus, oral's, ovatus, pectinophilus, plebeius, stercoris, thetaiotaomicron, uniformis, vulgates* and *xylanisolvens;* a *Prevotella* species selected from *bergensis, bivia, buccae, buccalis, copri, melaninogenica, oris, ruminicola, tannerae, timonensis* and *verorali; a Firmicutes* species selected from one or more *of Anaerotruncus, Acetanaerobacterium, Acetitomaculum, Acetivibrio, Anaerococcus, Anaerofilum, Anaerosinus, Anaerostipes, Anaerovorax, Butyrivibrio, Clostridium, Capracoccus, Dehalobacter, Dialister, Dorea, Enterococcus, Ethanoligenens, Faecalibacterium, Fusobacterium, Gracilibacter, Guggenheimella, Hespellia, Lachnobacterium, Lachnospira, Lactobacillus, Leuconostoc, Megamonas, Moryella, Mitsuokella, Oribacterium, Oxobacter, Papillibacter, Proprionispira, Pseudobutyrivibrio, Pseudoramibacter, Roseburia, Ruminococcus, Sarcina, Seinonella, Shuttleworthia, Sporobacter, Sporobacterium, Streptococcus, Subdoligranulum, Syntrophococcus, Thermobacillus, Turibacter* and *Weisella.* In some embodiments, the hegresin peptide variants exhibit antimicrobial activity against a bacterial species selected from: *Faecalibacterium prausnitzii* (A2-165, L2-6, M21/2 or SL3/3), a species of *Escherichia* (*E coli*)*, Shigella* (*dysenteriae*)*, Salmonella* (*typhi* or *enterica*)*, Erwinia, Yersinia* (*pestis*)*, Bacillus, Vibrio, Legionella* (*pneumophilia*)*, Pseudomonas* (*aeruginosa*)*, Neisseria* (*gonorrhea* or *meningitidis*)*, Bordetella* (*pertussus*)*, Helicobacter* (*pylori*)*, Listeria* (*monocytogenes*)*, Agrobacterium, Staphylococcus* (*aureus,* or MRSA), *Streptococcus* (*pyogenes* or *thermophilus*)*, Enterococcus, Clostridium* (*dificile* or *botulinum*)*, Corynebacterium (amycolatum), Mycobacterium (tuberculosis), Treponema, Borrelia (burgdorferi), Francisella, Brucella, Campylobacter (jejuni), Klebsiella (pneumoniae), Frankia, Bartonella, Rickettsia, Shewanella, Serratia, Enterobacter, Proteus, Providencia, Brochothrix, Bifidobacterium, Brevibacterium, Propionibacterium, Lactococcus, Lactobacillus, Pediococcus, Leuconostoc, Vibrio* (*cholera,* O139, or *vulnificus*)*, Haemophilus* (*influenzae*)*, Brucella* (*abortus*)*, Franciscella, Xanthomonas, Erlichia, Chlamydia (pneumoniae), Parachlamydia, Enterococcus* (*faecalis* or *faceim*)*,* Oenococcus and *Acinetoebacter* (*baumannii*)*.*

In some embodiments, the hegresin peptide variants exhibit antimicrobial activity against bacterial which are resistant to conventional antibiotics, further expanding the library of options available to the medical provider in addressing bacterial infections.

In some embodiments, the hegresin peptide variants exhibit antimicrobial activity against *Staphylococcus aureus* resistant to an antibiotic selected from methicillin, vancomycin-resistant and teicoplanin.

In some embodiments, the hegresin peptide variants exhibit antimicrobial activity against *Pseudomonas aeuroginosa* resistant to an antibiotic selected from cephalosporins (ceftazidime), carbapenems (imipenem or meropenem), fluoroquinolones, aminoglycosides (gentamicin or tobramycin) and colistin.

In some embodiments, the hegresin peptide variants exhibit antimicrobial activity against *Klebsiella* (eg, *pneumoniae*) resistant to carbapenem.

In an example, the first (host) cells are *Streptoccocus* (including *pneumonia* or *pyogenes*) resistant to an antibiotic selected from erythromycin, clindamycin, beta-lactam, macrolide, amoxicillin, azithromycin and penicillin.

In some embodiments, the hegresin peptide variants exhibit antimicrobial activity against *Salmonella* resistant to an antibiotic selected from ceftriaxone, azithromycin and ciprofloxacin.

In some embodiments, the hegresin peptide variants exhibit antimicrobial activity against *Shigella* resistant to an antibiotic selected from ciprofloxacin and azithromycin.

In some embodiments, the hegresin peptide variants exhibit antimicrobial activity against *mycobacterium tuberculosis* resistant to an antibiotic selected from isoniazid, rifampicin, fluoroquinolone, amikacin, kanamycin and capreomycin.

In some embodiments, the hegresin peptide variants exhibit antimicrobial activity against *Enterococcus* resistant to vancomycin.

In some embodiments, the hegresin peptide variants exhibit antimicrobial activity against *E. coli* resistant to an antibiotic selected from trimethoprim, itrofurantoin, cefalexin and amoxicillin.

In some embodiments, the hegresin peptide variants exhibit antimicrobial activity against *Clostridium* (*dificile*) resistant to an antibiotic selected from fluoroquinolone antibiotic and carbapenem.

In some embodiments, the hegresin peptide variants exhibit antimicrobial activity against *Neisseria gonnorrhoea* resistant to an antibiotic selected from cefixime (cephalosporin), ceftriaxone (cephalosporin), azithromycin and tetracycline.

In some embodiments, the hegresin peptide variants exhibit antimicrobial activity against *Acinetoebacter baumannii* resistant to an antibiotic selected from beta-lactam, meropenem and a carbapenem.

In some embodiments, the hegresin peptide variants exhibit antimicrobial activity against *Campylobacter* resistant to an antibiotic selected from ciprofloxacin and azithromycin.

Accordingly, in some embodiments, the hegrisin variants may prevent or treat diseases caused by these or other bacteria, including but not limited to, respiratory infections, ear infections, sinusitis, tonsillitis, urinary tract infections, prostate infections, sexually transmitted infection, gastrointestinal infections, skin infections, food poisoning, candidiasis, typhoid, cholera, etc. Therefore, in some embodiments, the hegrisin variants may be used as an active ingredient in a pharmaceutical composition for preventing or treating an infectious disease caused by the microorganisms.

### Compositions

In some embodiments, the hegrisin peptide variants are formulated into an antimicrobial composition. In some embodiments, the composition is a pharmaceutical composition containing a hegrisin peptide variant as an active ingredient. In some embodiments, a method for administering the composition to a subject in need thereof is provided. The method of administration is not particularly limited. In some embodiments, the composition may be administered intraarterially, intravenously, subcutaneously, intrarectally, intranasally, directly into muscle cells, or via any other parenteral route. In some embodiments, the composition may be administered orally (e.g., as a tablet, capsule, pill, suspension, liquid, a food composition), nasally, rectally, transdermally, or via injection.

In some embodiments, the hegresin peptide variant is combined with a pharmaceutically acceptable carrier or diluent to produce a pharmaceutical composition. The term "pharmaceutically acceptable carrier" refers to any pharmaceutical carrier that does not itself induce the production of antibodies harmful to the individual receiving the composition, and which can be administered without undue toxicity.

Pharmaceutically acceptable salts can also be present, e.g., mineral acid salts such as hydrochlorides, hydrobromides, phosphates, sulfates, and the like; and the salts of organic acids such as citrates, acetates, propionates, malonates, benzoates, and the like.

Suitable carriers and diluents include buffered, aqueous solutions, saline, dextrose, glycerol, isotonic saline solutions, for example phosphate-buffered saline, isotonic water, and the like and combinations thereof. In some embodiments, carriers may include propylene glycol, dimethyl isosorbide, and water, and even more particularly, phosphate buffered saline, isotonic water, deionized water, monofunctional alcohols and symmetrical alcohols. In some embodiments pharmaceutically acceptable carrier or diluent may be or contain a water soluble polymer, a carboxycellulose (e.g. carboxymethylcellulose), a collagen (e.g., a Type I collagen), a collagenous material comprising tropocollagen, a hyaluronan or derived-hyaluronic acid, and/or an oil (e.g., mineral oil). Suitable carriers can be large, slowly metabolized macromolecules such as proteins, polysaccharides, polylactic acids, polyglycolic acids, polymeric amino acids, and amino acid copolymers. The pharmaceutical compositions of this invention do not comprise a hegresin peptide variant or peptidomimetic in sterile water as the only vehicle.

In some embodiments, hegresin peptide variant or peptidomimetic, can be formulated in a particulate formulation one or a plurality of particles for selective delivery to the physiological region to be treated. In some embodiments, the particle can be, for example, a nanoparticle, a nanosphere, a nanocapsule, a liposme, a polymeric micelle, or a dendrimer. In some embodiments, the particle can be a microparticle. The nanoparticle or microparticle can comprise a biodegradable polymer.

In some embodiments, the compositions are isotonic with the fluids of the blood, and may have an osmolality of at least about 200 mOsmol/kg, preferably in the range of about 200 to about 350, or about 400 mOsmol/kg. The compositions may comprise, for example, sodium chloride, potassium chloride, calcium chloride and/or magnesium chloride.

In some embodiments, the composition is in the form of a foodstuff. The foodstuff can comprise a hegrisin peptide variant or peptidomimetic and a bulking agent, binding agent, flavorant, and/or lubricant. Bulking agents can include or exclude soymeal, soy powder, corn, cornmeal, and dried blood or hydrolyzed blood. Flavorants can include or exclude sugars (sucrose, glucose, mannose, dextrose, fructose, etc.), mint oils, cinnamon, terpenes, vanilla, caramel, cocoa, licorice, maltol, and glycerin. Binding agents can include or exclude gluten, guar gum, psyllium, potato starch, cornstarch, Xanthan gum, Carrageenan, Millet flour, gelatin, Almond flour, and Ground flaxseed. Lubricants can include or exclude silicone oil, plant oils (e.g., olive soil, cannola oil, corn oil, sesame oil, etc.), and animal fat.

In some embodiments, the hegrisin variants may also be used as an active ingredient of an antimicrobial food. In such embodiments, the hegrisin variants can be used in an antimicrobial food or feed additive because the hegrisin variants have superior antimicrobial activity against Gram-negative and Gram-positive bacteria, as well as fungi.

In such embodiments, the type of food is not particularly limited. Examples of the food to which the substance can be added include a drink or beverage, including an alcoholic beverage, meat, sausage, bread, biscuit, rice cake, chocolate, candy, snack, pizza, noodles, gum, soup, a diary product, such as yogurt or ice cream, etc. etc. In some embodiments, the food is a vitamin supplement and other health-functional food. The hegrisin variants may be added to a food as is or mixed together with other food ingredients. The adequate amount of the active ingredient may be determined depending on the purpose of use (*e.g.,* for prevention or treatment). In some embodiments, the hegrisin variants may be added in an amount of about 0.01 to about 50 wt.%, or about 0.1 to about 20 wt.%, or about 0.1 to about 10 wt.%, or about 0.1 to about 1 wt.%, based on the total weight of the food. All ranges are inclusive and combinable. In some embodiments, the amount of the active ingredient may be smaller than the above-described range. For example, a smaller amount may be used when the composition is used for health or hygiene or otherwise used for a long period of time. In some embodiments, a larger amount of the active ingredient may be used. For example, when there are no safety concerns.

In some embodiments, the hegrisin variants may be used as an active ingredient of an antimicrobial feed additive or feed composition, including any compound, preparation, mixture, or composition suitable for, or intended for intake by an animal such as a chicken, turkey, pig or swine, cow, sheep, horse, etc. In such embodiments, the hegrisin variants have provide antimicrobial activity against Gram-positive and Gram-negative bacteria. In some embodiments, the hegrisin variants may be added in an amount, including, e.g., about 0.01 to about 10.0%; about 0.05 to about 5.0%; or about 0.1 to about 1.0% (% meaning gram additive per 100 grams of feed). All ranges are inclusive and combinable.

In some embodiments, the hegrisin variants may also be used to preserve or hygienize antimicrobial feed because the hegrisin variants have superior antimicrobial activity against many bacteria such as *Campylobacter hepaticus* and *Clostridium perfringens,* major feed-borne pathogens. The hegrisin variants can be added directly to the animal feed in a treatment process of feed at levels of 0.01 to 10.0%; more particularly 0.05 to 5.0%; or 0.1 to 1.0% (% meaning gram additive per 100 grams of hygiene). All ranges are inclusive and combinable.

In some embodiments, the hegrisin variants may be used as an active ingredient in an antimicrobial cosmetic composition. The cosmetic composition may be in the form of a solution, powder, emulsion, lotion, spray, ointment, aerosol, cream, or foam. In some embodiments, the cosmetic composition may contain a carrier that is acceptable in a cosmetic formulation, additional active ingredients, or both. A "carrier that is acceptable in a cosmetic formulation" refers to a compound or composition already used in cosmetic formulations or a compound or composition to be developed, which lacks toxicity, instability, or irritability when applied to the skin of a subject. Examples of additional active ingredients include, but are not limited to steroids, salicylic acid, benzoyl peroxide, retinol, vitamin C, vitamin E, alpha hydroxy acids, dimethicone, and petrolatum.

As used herein, the term "skin" includes not only the face, but also the scalp and the entire body. For example, the cosmetic composition may be prepared as a shampoo, rinse, treatment, hair restorer, etc., for application to the scalp. And, for application to the entire body, the composition may be prepared as a body cleanser, soap, etc.

In some embodiments, the carrier may be contained in an amount of about 1 to about 99.99 wt %, or about 90 to about 99.99 wt %, based on the total weight of the cosmetic composition. For example, in some embodiments, the carrier may include an alcohol, oil, surfactant, fatty acid, silicone oil, humectant, moisturizer, viscosity modifier, emulsifier, stabilizer, sunscreen, UV absorbent, colorant, and/or fragrance, etc.

In some embodiments, the cosmetic composition may further compriseglycerin, butylene glycol, propylene glycol, polyoxyethylene hydrogenated castor oil, ethanol, triethanolamine, etc. In some embodiments, the composition may contain a trace amount of an antiseptic, fragrance, colorant, purified water, etc.

In some embodiments, the hegrisin variants may be used as an active ingredient of a hygiene product such as a wet wipe, hand sanitizer, mouthwash, oral antiseptic, toothpaste additive, etc. The hegrisin variants should be used in an amount that is effective for inhibiting microbial growth.

In some embodiments, the hegrisin variants may be used for cleaning, disinfecting or inhibiting microbial growth on any surface. Examples of surfaces, which may be contacted with the hegrisin variants include the surface(s) of manufacturing plants or equipment used therein, e.g., dairies, chemical or pharmaceutical process plants, water sanitation systems, oil processing plants, food processing plants, paper pulp processing plants, water treatment plants, and cooling towers. The hegrisin variants should be used in an amount that is effective for cleaning, disinfecting, or inhibiting microbial growth on the surface.

In some embodiments, the dosage of the composition will depend on the activity of the hegrisin variant, administration route, severity of the condition to be treated, condition and previous disease history of the patient, etc. However, starting with a lower dosage than is required to achieve the desired therapeutic effect and gradually increasing the dosage until the desired effect is achieved is within the knowledge of one skilled in the related art, and the specific administration dosage may be determined considering the age, sex, body type, and body weight. In some embodiments, the composition may be further processed before being formulated into a pharmaceutically acceptable pharmaceutical agent. For example, the composition may be pulverized or ground into particles. In some embodiments, depending on the desired effect, an effective dosage of the hegrisin peptide variants may be about 0.1 to about 10 mg/kg, about 1 to about 2 mg/kg, about 0.5 to about 1 mg/kg, or any dosage between the aforementioned dosages. In some embodiments, administration may be 1 to 10, 1 to 5, or 1 to 3 times a day. All ranges are inclusive and combinable.

In some embodiments, a pharmaceutical composition comprising a hegrisin variant may be prepared into a formulation of a single dosage form or a multiple dosage form using a pharmaceutically acceptable carrier and/or excipient according to a method typically employed by one of ordinary skill in the art. The formulation may be an oral formulation, such as a powder, granule, tablet, capsule, suspension, emulsion, syrup, aerosol, etc. The formulation may be formulated for external application such as an ointment, cream, etc., or any other pharmaceutical formulation such as a suppository, sterile solution for injection, etc. In some embodiments, the composition may further comprise a dispersant or stabilizer.

### Article of Manufacture

Articles of manufacturer are also provided, comprising a vessel containing a composition or formulation of the invention as described herein and instructions for use for the treatment of a subject. For example, in another aspect, the invention includes an article of manufacture comprising a vessel containing a therapeutically effective amount of one or more hegresin peptide variants or peptidomimetics, together with instructions for use, including use for the treatment of a subject. In some aspects the article of manufacture may comprise a matrix that comprises one or more hegresin variant peptides or peptidomimetics.

### EXAMPLES

### Strains, reagents, plasmids, enzymes, and growth media

Chemicals were obtained from Sigma-Aldrich Co. (St. Louis, MO). the 5% defibrinated horse blood, peptone and yeast extract, Difc tryptic soy agar (TSA) and tryptic soy broth (TSB) (Pancreatic digest of Casein 15g/L, Papaic digest of Soybean 5g/L, Sodium Chloride 5g/L, Agar 15g/L), LB Broth (Luria low salt), Nutrient Broth (Beef extract 3g/L, Peptone 5g/L), Brucella Broth (Tryptone 10 g/L, Peptone 10 g/L, Dextrose 1 g/L, Yeast Extract 2 g/L, Sodium Chloride 5.0 g/L, Sodium metabisulfite.0.1 g/L) were from Thermo Fisher Scientific Inc. (Pittsburgh, USA). Restriction enzymes, Phusion high-fidelity DNA polymerase, and T4 ligase were purchased from New England Biolabs (Ipswich, USA). *Escherichia coli* DH5a, *P. pastoris* X33 and vectors pCR-blunt and pPicZalpha were purchased from Invitrogen (San Diego, CA). Minimal dextrose (MD) medium, minimal methanol (MM) medium, buffered glycerol complex (BMGY) medium, buffered methanol complex (BMMY) medium, and fermentation Basal Salts medium (BSM) were prepared according to the manual of Pichia Expression kit (Life Technologies Corp. USA).

The bacterial strains *Campylobacter hepaticus* NCTC 13823 was purchased from National Collection of Type Center (NCTC, Salisbury, UK). The bacterial strains, including *Campylobacter jejuni* ATCC 12824, *Campylobacter coli* ATCC 49941, *Campylobacter fetus* ATCC 25936, *Campylobacter ureolyticusi* ATCC 33387, *Clostridium perfringens* ATCC 13124, *Clostridium perfringens* ATCC 10543, *Clostridium perfringens* ATCC 3626, *Clostridium perfringens* ATCC 27324, *Clostridium perfringens* ATCC 51880, *Staphylococcus aureus* ATCC *6538, Staphylococcus aureus* MRSA ATCC 43300, *Staphylococcus epidermidis* ATCC 14990, *Bacillus subtilis* ATCC 6633 and *Escherichia coli* ATCC 25922 were purchased from ATCC (Manassas, USA).

### Construction of expression plasmids

The Hegrisin variants were optimized according to codon usage bias and GC content of P. pastoris using Genscript's OptimumGen designing tool (Piscataway, NJ). The designed Hegrisin variants was synthesized by Eton Bioscience (Boston, USA) and were subcloned into pPicZalpha vector (Life Technologies Corp., USA). The resulting expression plasmid pPicZa was confirmed by restriction digestion and DNA sequencing (Eton Bioscience, USA).

### Yeast transformation and Screening of recombinant Pichia strains

The plasmid pPicZa was linearized with Pme I and then transformed into P. pastoris X33 by electroporation according to the manufacturer's instructions (Life Technologies Corp., USA). Transformants were screened on on YPD (1% yeast extract, 2% peptone, 2% glucose) plates containing 100 ug/ml Zerocin (Life Technologies Corp., USA). The positive recombinants were analyzed by genomic PCR with 5' AOX and 3' AOX primers. The recombinants identified by PCR were further screened in 125 mL shaken flasks. These strains were inoculated into 5 mL BMGY (1% yeast extract, 2% peptone, 1.34% YNB, 4× 10-5% biotin, 1% glycerol and 100 mM potassium phosphate, pH 6.0) and cultured for 24 h at 30 °C in 50 mL shaker flasks in a shaking incubator (250 rpm). After culture reaches an OD600 = 6, one mL culture was transferred to 125 mL shaker flask containing 10 mL BMMY (1% yeast extract, 2% peptone, 1.34% YNB, 4×10-5% biotin, 0.5% methanol and 100 mM potassium phosphate, pH 6.0) and cultured for 24 h at 30 °C (250 rpm). The enzyme expression was induced by adding 100% methanol to a final concentration of 0.5% methanol. The supernatant was collected by centrifugation at 12,000 rpm for 10 min (at 4 °C) for antimicrobial activity assay. The expressed peptides were analyzed by Tricine-SDS-PAGE.

### Purification of expressed peptides

The fermentation supernatant was precipitated with 40-45% ammonium sulfate. The precipitated peptides were centrifuged at 15000 xg for 30 min. The pellets were re-suspended with deionized water and purified a Sephadex G-25 column and eluted with deionized water at a rate of 0.5 ml/min. The peak absorbance fractions were pooled for subsequent antimicrobial assays.

### Antimicrobial activity assay

The antimicrobial activity of purified Hegrisin variants were analyzed using the inhibition zone assay. Test strains of *C*. *hepaticus* NCTC 13823 was streaked on BB plates (Brucella broth + 1.5% agar supplemented with 5% defibrinated horse blood (HBA) and cultured at 37 °C under microaerobic conditions (created using Campygen 3.5L gas generation packs (Oxoid) in an anaerobic jar, for 96 h. The *C*. *hepaticus* cells were harvested from an BB plate and suspended into 5ml BB. The 0.5 ml bacterial suspension was spread on the surface of BB plate. The 5 mm holes were punched with a glass capillary and were filled with 50 µL of solution containing 10 µg Hegrisin variants. Ampicillin (1µg) was used as a positive control and sterile PBS was used a negative control. After incubation under microaerobic conditions at 37°C for 48 h, the zones of growth inhibition were measured.

### The minimal inhibitory concentration (MIC) assay

Minimal Inhibitory Concentration assays (MIC, expressed as µg/mL) against different microorganisms were performed according to the protocol described in the CLSI: (Methods for Dilution Antimicrobial Susceptibility Testing for Bacteria; Approved Standard - Eleventh Edition (2012). The tested bacteria *C*. *hepaticus* NCTC 13823 and *C. jejuni* ATCC 12824 strains were streaked on BB agar and grown microaerobically at 37°C for 48 h, then the cells were harvested in 2mL BB media and diluted in the same medium to the appropriate concentration (OD600 nm =0.05). Then, 50µL peptides with various concentrations was added to 50 µL diluted culture fluid containing testing strains, resulting in a total volume of 100 µL. The 96-well microplates were incubated at 37°C for 48 h, and absorbance at 600 nm were taken to determine MIC.

The tested bacteria *C. perfringens* ATCC 13124, *C. perfringens* ATCC 10543, *C*. *perfringens* ATCC 3626, *C. perfringens* ATCC 27324, *C. perfringens* ATCC 51880 strains were streaked on RCM agar and grown microaerobically at 37°C for 24 h, then the cells were harvested in 2mL RCM media and diluted in the same medium to the appropriate concentration (OD600 nm =0.05). Then, 50µL peptides with various concentrations was added to 50 µL diluted culture fluid containing testing strains, resulting in a total volume of 100 µL. The 96-well microplates were incubated at 37°C for 15 h, and absorbance at 600 nm were taken to determine MIC.

The tested bacteria *S. aureus* ATCC 6538, *S. aureus,* MRSA ATCC 43300, *S. epidermidis* ATCC 14990, and *B. subtilis* ATCC 6633 were grown to OD600=0.5 at 37°C in TSB or LB broth. The bacterial cultures were diluted with medium to 10⁴-10⁶ CFU/mL. Then, 50µL peptides with various concentrations was added to 50 µL diluted culture fluid containing testing strains, resulting in a total volume of 100 µL. The 96-well microplates were incubated at 37°C for 15 h, and absorbance at 600 nm were taken to determine MIC.

The MIC value was defined as the lowest peptide concentration that completely prevented growth using a microtiter optical plate reader.

### Hemolytic assay

The hemolytic toxicity of Hegrisin variants Heg01, Heg03, Heg04, Heg05, Heg11 and Heg21 were determined using 2% suspensions of human erythrocytes (Medix Biochemica, St Louis, USA). The Hegrisin variants were diluted to concentrations of 250, 125, 62.5, 32, 16, 8, 4, 2, 1, and 0.5 µg/ml. The 100 µL of peptide solution and 100 µL of red blood cell suspension was mixed and added to the wells of a 96-well plate. The PBS was used as negative control and Triton X-100 was used as a positive control.

The samples were incubated at 37 °C for 60 min and gently stirred during the incubation period. Then, the samples were centrifuged at 2000 rpm for 5 min. A total of 100 µL of the supernatant in each well was transferred to a new 96-well plate and absorbance was measured at 490 nm using a microplate reader (Molecular Devices, USA).

### Example 1

### Designed Hegrisin variants

Hegrisin is a defensin-like antimicrobial peptide consisting of 38 amino acids and having a molecular weight of 4111.8 Da. To increase antimicrobial activity against Gram-negative bacteria *C*. *hepaticus* and Gram-positive bacteria such as *C. perfringens,* twenty (20) representative hegrisin peptide variants were designed and evaluated. The quality estimate ranged between 1 and 2 with higher values for better models.

The sequences and activities of Hegrisin and variants were shown in Table 1.

**TABLE 1**

| SEQ ID NO | Peptides | Sequence | Activity to *C. hepaticus* |
|---|---|---|---|
| SEQ ID NO 1 | Hegrisin | GFGCTIWGGNDKPCHRHCKSIKGYKGGYCKVGGVCKCY | 1 |
| SEQ ID NO 2 | Heg02 | GFGCTIWGGNDDPCHRHCKSIKGYKGGYCKVGGVCKCY | 1 |
| SEQ ID NO 3 | Heg03 | GFGCTIWGGNDEPCHRHCKSIKGYKGGYCKVGGVCKCY | 2 |
| SEQ ID NO 4 | Heg04 | GFGCTIWGGNDEPCHRHCKSIKGYKGGYCKVGGICKCY | 2 |
| SEQ ID NO 5 | Heg05 | GFGCTIWGGNDEPCHRHCKSIKGYKGGYCKFGGVCKCY | 2 |
| SEQ ID NO 6 | Heg06 | GFGCTIWGGNDEPCHQHCKSIKGYKGGYCKVGGVCKCY | 1 |
| SEQ ID NO 7 | Heg07 | GFGCTIWGGNDEPCHNHCKSIKGYKGGYCKVGGVCKCY | 1 |
| SEQ ID NO 8 | Heg08 | GFGCTIWGGNDEPCHRHCKSIKGYKGGYCKFGGICKCY | 1 |
| SEQ ID NO 9 | Heg09 | GFGCTIWGGNDEPCHRHCKSIKGYKGGYCKLGGICKCY | 1 |
| SEQ ID NO 10 | Heg10 | GFGCTIWGGNDEPCHRHCKSIRGYKGGYCKVGGVCKCY | 1 |
| SEQ ID NO 11 | Heg11 | GFGCTIWGGNDEPCHRHCKSIRGYRGGYCKVGGVCKCY | 2 |
| SEQ ID NO 12 | Heq12 | GWSCNIWNGNDEPCHQHCKSIRGYRGGYCKFGGVCKCY | 1 |
| SEQ ID NO 13 | Heg13 | GWSCNIWNGNDEPCHQHCKSIRGYRGGYCKVGGVCKCY | 1 |
| SEQ ID NO 14 | Heg14 | GWSCNIWNGNDEPCHQHCKSIKGYKGGYCKVGGVCKCY | 1 |
| SEQ ID NO 15 | Heg15 | GWSCNIWNGNDEPCHRHCKSIKGYKGGYCKVGGVCKCY | 1 |
| SEQ ID NO 16 | Heg16 | GWSCNIWNGNDEPCHNHCKSIKGYKGGYCKVGGVCKCY | 1 |
| SEQ ID NO 17 | Heg17 | GWSCNIWNGNDEPCHRHCKSIKGYKGGYCKFGGICKCY | 1 |
| SEQ ID NO 18 | Heg18 | GWSCNIWNGNDEPCHKHCKSIRGYRGGYCKFGGICKCY | 1 |
| SEQ ID NO 19 | Heg19 | GWSCNIWNEDDLRCHNHCKSIRGYRGGYCKFGGICKCY | 1 |
| SEQ ID NO 20 | Heg20 | GWSCNIWNEDDLRCHQHCKSIKGYKGGYCKVGGVCKCY | 1 |
| SEQ ID NO 21 | Heg21 | GWSCGFFGGNDEPCHQHCKSIRGYRGGYCKVGGICKCY | 2 |

The sequences of hegrisin variants are shown in **Table 1** along with their corresponding antimicrobial activities relative to the activity of hegrisin. An activity of 1 corresponds to the activity of hegrisin. An activity of 2 corresponds to an activity that is better than that of hegrisin.

The hegresin peptide variants of the formula
GX₁X₂CX₃X₄X₅X₆X₇X₈DX₉X₁₀CHX₁₁HCKSIX₁₂GYX₁₃GGYCKX₁₄GGX₁₅CKCY (SEQ ID NO: 23), wherein X₆ is G, X₇ is G, and X₈ is N, wherein X₆ is G, X₇ is G, and X₈ is N exhibited the highest antimicrobial activity.

### Example 2

### Evaluation of antimicrobial activity

The Hegrisin variants were expressed in *P. pastoris* X33. The supernatant was collected by centrifugation at 12,000 rpm for 10 min (at 4 °C). The fermentation supernatant was precipitated with 40-45% ammonium sulfate. The precipitated peptides were centrifuged at 15,000 xg for 30 min. The pellets were re-suspended with deionized water and purified with Sephadex G-25 columns.

The antimicrobial activity of purified Hegrisin variants were analyzed using the inhibition zone assay. Test strains of *C*. *hepaticus* NCTC 13823 was streaked on BB plates (Brucella broth + 1.5% agar supplemented with 5% defibrinated horse blood (HBA) and cultured at 37 °C under microaerobic conditions (created using Campygen 3.5L gas generation packs (Oxoid) in an anaerobic jar, for 96 h. The C. hepaticus cells were harvested from an BB plate and suspended into 5ml BB. The 0.5 ml bacterial suspension was spread on the surface of BB plate. The 5 mm holes were punched with a glass capillary and were filled with 50 µL of solution containing 10 µg Hegrisin variants. Ampicillin (1µg) was used as a positive control and sterile PBS was used a negative control. After incubation under microaerobic conditions at 37°C for 48 h, the zones of growth inhibition were measured.

### Example 3

### Measuring Minimal Inhibitory Concentration

The Hegrisin variants (SEQ ID NO: Heg03, Heg04, Heg05, Heg11 and Heg21) with better antimicrobial activity were selected for further analysis. The hegrisin variants were tested using a minimal inhibitory concentration assay (MIC, expressed as µl/mL) following the protocol described in the Clincial and Laboratory Standards Institute (CLSI) Methods for Dilution Antimicrobial Susceptibility Testing for Bacteria; Approved Standard - Eleventh Edition (2012) against the following microorganisms: *C. hepaticus* NCTC 13823, *C. perfringens* ATCC 13124, *S. aureus* ATCC 6538, and *S. aureus* MRSA ATCC 43300. The results are provided in Table 2.

Compared to the wildtype hegrisin (SEQ ID NO: 1), the hegrisin variants Heg03 (SEQ ID NO: 3), Heg04 (SEQ ID NO: 4) and Heg21 (SEQ ID NO: 21) exhibited improved antimicrobial activity (i.e., lower MIC values) against the bacteria *C*. *hepaticus* NCTC 13823, *C*. *perfringens* ATCC 13124, *S. aureus* ATCC 6538, and *S. aureus* MRSA ATCC 43300. The Heg05 (SEQ ID NO: 5) and Heg11 (SEQ ID NO: 11) exhibited improved antimicrobial activity against the bacteria *C*. *hepaticus* NCTC 13823, *C. perfringens* ATCC 13124, *S. aureus* ATCC 6538, but not to *S. aureus* MRSA ATCC 43300.

The Minimal inhibitory concentration (MIC) for hegrisin and the hegrisin variants against Gram-negative and Gram-positive bacteria is shown in **Table 2.**

**Table 2 Minimal inhibitory concentration (MIC) of Hegrisin variants against Gram-positive and Gram-negative bacteria**

| Microbe | Minimal inhibitory concentration (MIC) (µg/ml) | | | | | |
|---|---|---|---|---|---|---|
| | SEQ ID NO | | | | | |
| | Heg01 | Heg03 | Heg04 | Heg05 | Heg11 | Heg21 |
| *C. hepaticus* NCTC 13823 | 6.80 | 1.03 | 1.28 | 3.60 | 3.40 | 1.65 |
| *C. perfringens* ATCC 13124 | 4.10 | 0.32 | 1.30 | 1.80 | 1.70 | 0.83 |
| *S. aureus* ATCC 6538 | 2.50 | 0.88 | 1.90 | 1.95 | 1.65 | 1.03 |
| *S. aureus* MASA ATCC 43300 | 1.67 | 1.17 | 1.27 | 2.60 | 2.20 | 1.37 |

### Example 4

### Evaluation of Antimicrobial Activity of Heg03 peptide

One Hegrisin variant (SEQ ID NO: 3; Heg03) with best antimicrobial activity was expressed and purified. The Minimal Inhibitory Concentration (MIC) was determined to test for its antimicrobial activity following the CLSI guidelines.

The Heg03 peptide were tested against the following strains: *C. hepaticus* NCTC 13823, *C. jejuni* ATCC 12824, *C*. *coli* ATCC 49941, *C. fetus* ATCC 25936, *C*. *ureolyticusi* ATCC 33387, *C. perfringens* ATCC 13124, *C. perfringens* ATCC 10543, *C. perfringens* ATCC 3626, *C. perfringens* ATCC 27324, *C. perfringens* ATCC 51880, *S. aureus* ATCC 6538, *S*. *aureus* MRSA ATCC 43300, *S. epidermidis* ATCC 14990, *B. subtilis* ATCC 6633 and *E. coli* ATCC 25922.

The results show Heg03 peptide had strong activity against Gram-negative bacteria *C*. *hepaticus* NCTC 13823, Gram positive bacteria *C. perfringens* ATCC 13124, *C*. *perfringens* ATCC 10543, *C. perfringens* ATCC 3626, *C. perfringens* ATCC 27324, *C*. *perfringens* ATCC 51880, *S. aureus* ATCC 6538, *S. aureus* MRSA ATCC 43300, *S. epidermidis* ATCC 14990, *B. subtilis* ATCC 6633. The Heg03 peptide has activity to *C. jejuni* ATCC 12824, but no activity against *C*. *coli* ATCC 49941, *C. fetus* ATCC 25936, *C*. *ureolyticusi* ATCC 33387 and *E. coli* 25922 (Table 3). The Minimal inhibitory concentration (MIC) for hegrisin variants Heg03 against Gram-negative and Gram-positive bacteria is shown in **Table 3.**

**Table 3**

| Microbe | Minimal inhibitory concentration (MIC) (µg/ml) |
|---|---|
| | Heg03 |
| *C. hepaticus* NCTC 13823 | 1.03 |
| *C. jejuni* ATCC 29428 | 41.0 |
| *C. perfringens* ATCC 13124 | 0.32 |
| *C. perfringens* ATCC 10543 | 0.22 |
| *C. perfringens* ATCC 3626 | 0.52 |
| *C. perfringens* ATCC 27324 | 0.72 |
| *C. perfringens* ATCC 51880 | 0.42 |
| *S. aureus* ATCC 6538 | 0.88 |
| *S. aureus* ATCC 25923 | 2.03 |
| *S. aureus* MRSA ATCC 43300 | 1.17 |
| *S. epidermidis* ATCC 14990 | 0.31 |
| *B. subtilis* ATCC 6633 | 0.46 |
| *C. coli* ATCC 49941 | >250.0 |
| *C. fetus* ATCC 25936 | >250.0 |
| *C. ureolyticus* ATCC 25936 | >250.0 |
| *E. coli* ATCC 25922 | >250.0 |

### Example 5

### Hemolytic activity of Hegrin variant peptides

For hemolysis assays, human erythrocytes were obtained from healthy donors, washed 3 times using sterilized PBS, and resuspended to a concentration of 2% (v/v) with PBS. The hegrisin and Heg variants Heg03, Heg04, Heg05, Heg1 1and Heg21 were diluted to concentrations of 250, 125, 62.5, 32, 16, 8, 4, 2, 1, and 0.5 µg/ml. A 100 µL portion of each peptide solution was mixed with a 100 µL red blood cell suspension (Medix Biochemica, St Louis, USA) and then added to the wells of a 96-well plate. PBS was used as negative control and Triton X-100 was used as a positive control. The samples were incubated at 37°C for 60 minutes and gently stirred during the incubation period. Then, the samples were centrifuged at 2000 rpm for 5 minutes. A total of 100 µL of the supernatant in each well was transferred to a new 96-well plate and absorbance was measured at 490 nm using a microplate reader (Molecular Devices, USA).

As shown in FIG. 1, hegrisin and the Hag variants Heg03, Heg04, Heg05, Heg11and Heg21 exhibited no significant lysis of the human cells at any concentration of peptides tested (0.5-250 µg/ml). By contrast, plectasin exhibited lysis at nearly all concentrations, and the cell lysis increased significantly as the concentration was increased from 62.5 µg/ml to 250 µg/ml.

As described above, the antimicrobial peptide hegrisin variants exhibit remarkable antibacterial effects against Gram-negative and Gram-positive bacteria without harmful side effects to human cells. The hegrisin variants are effective active ingredients for feed additives, food preservatives, cosmetics, and/or pharmaceutical compositions.

A recitation of a range of values herein is merely intended to serve as a shorthand method of referring individually to each value falling within the range, unless otherwise indicated herein, and each separate value is incorporated into the specification as if it were individually recited herein. Thus, for example, a component provided in 1-3 grams refers to the component being provided in 1, 2, or 3 grams. As will be understood by one skilled in the art, ranges disclosed herein encompass any and all possible subranges and combinations of subranges thereof. Any listed range can be easily recognized as sufficiently describing the same range being broken down into at least equal halves, thirds, quarters, fifths, tenths, etc. As a nonlimiting example, each range discussed herein can be readily broken down into a lower third, middle third and upper third, etc. As will also be understood by one skilled in the art, language such as "up to," "at least," "greater than," "less than," and the like include the number recited and refer to ranges which can be subsequently broken down into subranges as discussed above.

As used herein, the use of examples, or exemplary language (e.g., "such as"), is intended to illuminate the embodiments and does not pose a limitation on the scope of the claims unless otherwise stated. No language in the specification should be construed as indicating any non-claimed element as essential.

As used herein, the terms "about" and "substantially" will be understood by persons of ordinary skill in the art and will vary to some extent depending upon the context in which it is used. If there are uses of the term which are not clear to persons of ordinary skill in the art, given the context in which it is used, "about" and "substantially" will mean up to plus or minus 10% of the particular term.

Exemplary embodiments of the methods are described above in detail. The methods are not limited to the specific embodiments described herein, but rather, steps of the method may be utilized independently and separately from other steps described herein. All methods described herein can be performed in any suitable order unless otherwise indicated herein or otherwise clearly contradicted by context.

The foregoing embodiments are provided to aid in the understanding of the present disclosure, the true scope of which is set forth in the appended claims. This written description uses examples to disclose the present embodiments, including the best mode, and also to enable any person skilled in the art to practice the present embodiments, including performing any methods. The patentable scope of the present embodiments is defined by the claims, and may include other examples that occur to those skilled in the art. Such other examples are intended to be within the scope of the claims if they have elements that do not differ from the literal language of the claims, or if they include equivalent elements with insubstantial differences from the literal language of the claims.

## Claims

1. An antimicrobial peptide comprising or consisting of an amino acid sequence having the following sequence:
GX₁X₂CX₃X₄X₅X₆X₇X₈DX₉X₁₀CHX₁₁HCKSIX₁₂GYX₁₃GGYCKX₁₄GGX₁₅CKCY (SEQ ID NO: 22),
wherein X₁ is F or W;
X₂ is G or S;
X₃ is T, N or G;
X₄ is I or F;
X₅ is W or F;
X₆ is G or N;
X₇ is G or E;
X₈ is N or D;
X₉ is E, L, or D;
X₁₀ is P or R;
X₁₁ is R, Q, N, or K;
X₁₂ is K or R;
X₁₃ is K or R;
X₁₄ is V, F, or L; and
X₁₅ is V or I.

2. The antimicrobial peptide of claim 1, wherein X₆ is G, X₇ is G, and X₈ is N.

3. The antimicrobial peptide of any of claims 1 to 2, wherein the amino acid sequence is selected from the group consisting of SEQ ID NOs: 2-21.

4. The antimicrobial peptide according to any of claims 1 to 3, wherein the antimicrobial peptide has antimicrobial activity against Gram-negative bacteria and/or Gram-positive bacteria,
preferably wherein the Gram-negative bacteria are selected from the group consisting of *Campylobacter hepaticus* and *Campylobacter jejuni* and/or preferably wherein the Gram-positive bacteria are selected from the group consisting of *Clostridium perfringens, Staphylococcus aureus, Staphylococcus aureus* MRSA, *Staphylococcus epidermidis, Bacillus subtilis.*

5. An antimicrobial peptide composition comprising the antimicrobial peptide of any of claims 1 to 4 as an active ingredient.

6. A pharmaceutical composition comprising the antimicrobial peptide of any of claims 1 to 4 as an active ingredient.

7. An antimicrobial food additive comprising the antimicrobial peptide of any of claims 1 to 4 as an active ingredient.

8. A cosmetic composition comprising the antimicrobial peptide of any of claims 1 to 4 as an active ingredient.

9. A hygiene product comprising the antimicrobial peptide of any of claims 1 to 4 as an active ingredient.

10. Use of the antimicrobial peptide of any of claims 1 to 4 as an active ingredient in an antimicrobial peptide composition, preferably wherein the antimicrobial peptide composition is a pharameceutical composition.

11. Use of the antimicrobial peptide of any of claims 1 to 4 as an active ingredient in an antimicrobial food additive.

12. Use of the antimicrobial peptide of any of claims 1 to 4 as an active ingredient in a cosmetic composition.

13. Use of the antimicrobial peptide of any of claims 1 to 4 as an active ingredient in a hygiene product.

14. The antimicrobial peptide of any of claims 1 to 4 for use in a method of treating an infectious disease caused by bacteria, preferably wherein the antimicrobial peptide is of the amino acid sequence of SEQ ID NO: 3 or SEQ ID NO: 4 or SEQ ID NO: 5 or SEQ ID NO: 11 or SEQ ID NO: 18 or SEQ ID NO: 21.

15. Method for producing a pharmaceutical composition comprising the antimicrobial peptide of any of claims 1 to 4 as an active ingredient compsirsing the steps of
- providing the antimicrobial peptide of claims 1 to 4
- combining the antimicrobial peptide of claims 1 to 4 with a pharmaceutically acceptable carrier or diluent.
